# EUROPEAN PATENT APPLICATION

(11) **EP 0 872 227 A2**
(43) Date of publication of application: **21.10.1998**
(21) Application number: 98300415.1
(22) Date of filing: 21.01.1998
(51) Int. Cl.: A61G 11/00, A61F 7/00

(54) **Proximity sensing infant warmer**

(30) Priority: 18.03.1997 US 820275
(71) Applicant: OHMEDA INC., Liberty Corner, New Jersey 07938-0804 (US)
(72) Inventor: Sweeney, Stephen J., Sykesville, Maryland 21784 (US); Spencer, Timothy A., New Windsor, Maryland 21776 (US)
(74) Representative: Hedley, Nicholas James Matthew

(57) **Abstract**

An infant care center having a overheat radiant heater that directs a pattern of infrared energy towards an infant bed to warm an infant positioned on the bed. A proximity sensor is located so as to detect an intrusion into the pattern of infrared radiation, such as when an attending person enters the pattern of radiation to carry our some function on the infant. The proximity sensor detects that intrusion by a caregiver and adjusts the intensity of the infrared radiation emitted by the heater. In the preferred embodiment, the radiant heater is turned off when the caregiver is in the pattern of infrared radiation to avoid discomfort to the caregiver.

## Description

The present invention relates to infant care centers of the type that include a support or bed for the infant as well as include an overhead heating unit that directs radiant energy toward the infant for heating that infant.

In such infant care centers, it is common to position radiant heaters above the infant and such heaters direct the infrared electromagnetic radiation toward the infant. The amount of radiation directed toward the infant is carefully controlled such that the radiation that actually reaches the infant is known and cannot cause harm to the infant. The infrared radiation forms a pattern or zone of radiation between the heater and the infant bed and, obviously, anything that enters that pattern is impinged upon by the infrared radiation.

One difficulty with such systems is, however, that the attendant caregiver, such as a nurse or physician, will occasionally attend to the infant to administer some care to that infant and in order to carry out such work, the caregiver must enter the pattern of the infrared radiation being supplied to the infant.

Since that radiation is in the infrared spectra, any part of the attending personnel that enters the pattern of radiation is immediately heated, causing discomfort to the personnel, sometimes quite severe since the point of intrusion by the personnel may be relatively close to the source. That is a bother and an annoyance to the personnel.

Obviously, one way to prevent such problem is for the personnel to manually disable the source of radiation before attending to the infant. In such case, however, it is possible for the caregiver to complete the tasks with the infant and inadvertently not turn the heater back on, thereby depriving the infant of needed warmth. In addition, it is often necessary to continue some heat to the infant even when the caregiver is carrying out some intervention rather that totally deprive the infant of heat during that period of time.

Another possible means of alleviating the problem is shown in U.S. Patent 4,832,029 where various patterns of radiation are established in order to create a zone where there is little or no harmful radiation that could impinge upon the attending personnel.

According to one aspect of the present invention, an infant care center comprises:
a standing frame member;
a generally planar infant bed affixed to said standing frame member and adapted to underlie an infant;
an infrared heater mounted to said standing frame member above said infant bed, said infrared heater adapted to radiate electromagnetic radiation in the infrared spectrum at an intensity forming a pattern of infrared radiation directed toward said infant bed;
a proximity sensor to detect an intrusion into said pattern of the infrared energy; and
means responsive to the detection of an intrusion by said proximity sensor to change the intensity of the infrared radiation radiated by said infrared heater.

The infant care center of the present invention includes a proximity sensing device that is activated when something, such as a portion of the attending personnel, intrudes into the pattern of infrared radiation directed toward the infant bed. When such intrusion is detected, a control device automatically takes corrective action to prevent, or at least alleviate, any distress to the attending personnel.

The proximity sensor may be located at or near the heater and senses the proximity of some blockage of its beam. Such proximity sensors are typically infrared themselves or ultrasonic and are commercially available. the proximity sensors have a pattern of recognition and any movement within that pattern causes the proximity sensor to recognize the existence of an intrusion. Typical commercial devices turn on a light or another appliance and may have a built in timer that turns the light back off when the intrusive body has been removed from the recognition pattern.

According to a further aspect of the present invention, an apparatus for heating an infant comprises:
an infant bed adapted to underlie an infant;
an infrared heater assembly mounted above said infant bed, said infrared heater assembly adapted to direct electromagnetic radiation in the infrared spectrum at an intensity toward said infant bed in a known pattern,
a proximity sensor adapted to sense the intrusion of a body into said known pattern of infrared radiation directed by said infrared heater assembly toward said infant bed; and
a controller means to control said infrared heater assembly to change the intensity of the infrared radiation directed by said infrared heater assembly toward said infant bed when said proximity sensor senses an intrusion.

According to yet a further aspect of the present invention, a method of providing heat to an infant positioned upon an infant bed comprises the steps of:
locating an infrared emitter above the infant bed;
directing the infrared radiation emitted from the infrared emitter in a pattern toward the infant bed;
sensing the intrusion of a body into the pattern of radiation directed generally toward infant bed to warm the infant; and
controlling the intensity of the infrared radiation from the infrared emitter in response to the sensed intrusion of the body.

In carrying out the present invention, therefore, the proximity sensor may be focused and/or located such that its pattern of recognition may be coextensive with the pattern of infrared radiation that is created by the radiant heater. Thus, whenever any part of the attending personnel intrudes upon the pattern of infrared radiation, the proximity sensor will immediately pick up the intrusive movement and take the corrective action. That corrective action may be to turn the radiant heater off completely or to reduce its intensity to a lower level so that some heating is still available to the infant. Accordingly, in accordance with conventional sensors, the radiant heater can be turned back on automatically when that intrusive body has been removed from the location with or without a time delay to prevent the radiant heater from being cycled too repetitively.

Thus, the proximity sensor of the present invention will automatically turn off or reduce the intensity of the radiant heater when any attending personnel intervene to work on the infant and when the personnel have completed the particular task, the heater will automatically be returned to its original intensity to provide warmth to the infant.

As a preferred embodiment of the invention, the proximity sensor may only lower the intensity of the infrared radiation emitted by the overhead heater so that the attending personnel are protected from the full effect of the infrared radiation yet some radiation continues to reach the infant to provide some heat. As a still further embodiment, the radiant overhead heater may comprise a plurality of heaters and each have a proximity sensor such that only those heaters that provide radiation directly impinging on the intrusive body are turned off or lowered and those heaters that do not sense the intrusive body continue to provide the heat to the infant.

Embodiments of the invention will now be described, by way of example, reference being made to the Figures of the accompanying diagrammatic drawings in which:-
FIG. 1 is an isometric view of an infant care center that incorporates the present invention;
FIG. 2A is a schematic view of an infant care center showing a single proximity sensor used in accordance with the present invention;
FIG. 2B is a schematic view of an infant care center utilizing a plurality of proximity sensors in carrying out the present invention;
FIG. 2C is a schematic view of the embodiment of FIG 2B where in a caregiver has entered the pattern of recognition of one or more proximity sensors; and
FIG. 3 is a isometric view of an overhead radiant heater having a plurality of heaters and proximity sensors in accordance with the present invention.

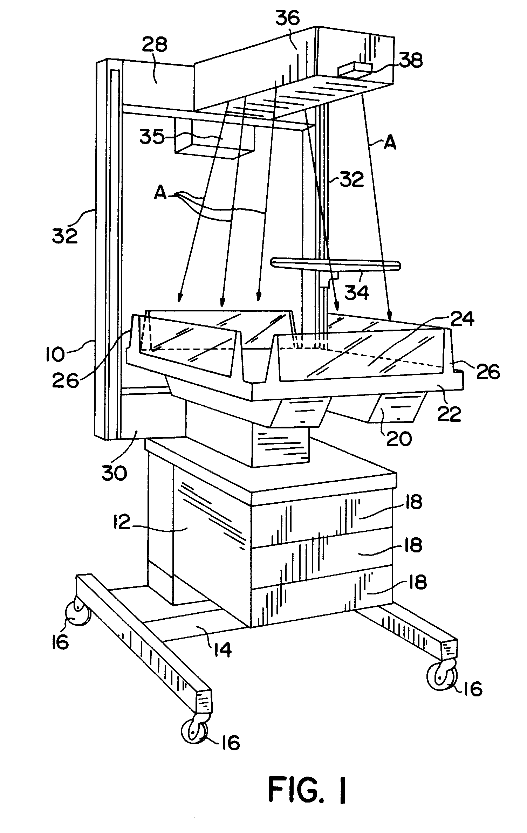

Referring now to FIG. 1, there is shown an isometric view of an infant care center having a conventional heater mechanism. As shown, the infant care center includes a frame 10 which provides a free standing unit for the infant care center. The frame 10 is supported upon a cabinet 12 which, in turn, is mounted upon a base 14 having wheels 16 so that the infant care center is easily movable. The cabinet 12 may also include one or more drawers 18 for containing items for attending to the infant.

An infant pedestal 20 is mounted atop of the cabinet 12 and on which is located an infant bed 22 which underlies an infant positioned thereon. Pedestal 20 is the main support for infant bed 22. The infant bed 22 has a generally planar upper surface 24 with appropriate cushioning material for comfort of the infant and further may be surrounded by guards 26, generally of a clear plastic material, and which contain the infant on the planar upper surface 24. Generally, the guards 26 are removable and/or releasable for complete access to the infant.

Frame 10 includes upper and lower cross members 28 and 30, respectively, joining a pair of vertical struts 32 and which vertical struts 32 may provide a means of support for other structural parts such as a shelf 34.

Mounted on the upper cross member 28 may be a control module 35 for containing the various electrical controls to operate the infant care center. In addition, a heater 36 is mounted to the upper cross member 28. As will be noted, the location of the heater 36 is such as to be above the infant bed 22. The heater 36 is focused so as to provide a footprint on and around the infant to optimize the amount of heat directed upon the infant. Various types of focusable heaters are available for such application, examples of which may be a Calrod focused heater of about 500-600 watts, or a corrugated foil heater such as is shown in and described in U.S. Patent 5,474,517. Preferably, the footprint of heat directed by the heater 36 to the infant bed 22 is generally rectangular and uniformly covers the infant bed 22.

As can be seen, a pattern of infrared radiation, indicated by the arrows A, extends from the heater 36 to the infant bed 22 and thus, any portion of an attending caregiver that enters that zone or pattern is impinged upon by the infrared radiation and can cause heating and discomfort to the person. In general the caregiver is bending over the infant to attend to that infant such that the head of the caregiver is directly in the pattern of infrared radiation and, due to the height of the person, the head is relatively close to the heater 36 such that considerable discomfort can be experienced.

As also shown in FIG. 1, a proximity sensor 38 is mounted to the heater 36 although it may be mounted elsewhere as long as the purpose of the present invention is accomplished. The proximity sensor 38 may be of the infrared type, ultrasonic type or other type and, in any event, is located such that its recognition pattern is coextensive with the pattern of infrared radiation from heater 36. By that is meant, the proximity sensor 38 itself emits a pattern of radiation and recognizes when that pattern has been intruded upon. The proximity sensor 38 thus takes some action or generates a signal based upon the recognition of an intrusion into its pattern of recognition.

That pattern of recognition of the proximity sensor 38 may be coextensive with the infrared pattern such that any intrusion into the pattern of infrared radiation will be detected by the proximity detector 38 or, alternatively, the pattern of recognition of the proximity detector may be slightly wider that the pattern of infrared radiation such that the proximity sensor 38 will be able to take the corrective action before the intrusion by the caregiver actually invades the pattern of infrared radiation.

The actual positioning of the proximity sensor 38 may depend upon the particular pattern desired by the user and the distance from the infant bed 22. The conventional proximity sensors can also be focused so as to attain the optimum of sensitivity for objects that enter into the recognition pattern between the infant bed 22 and the heater 36.

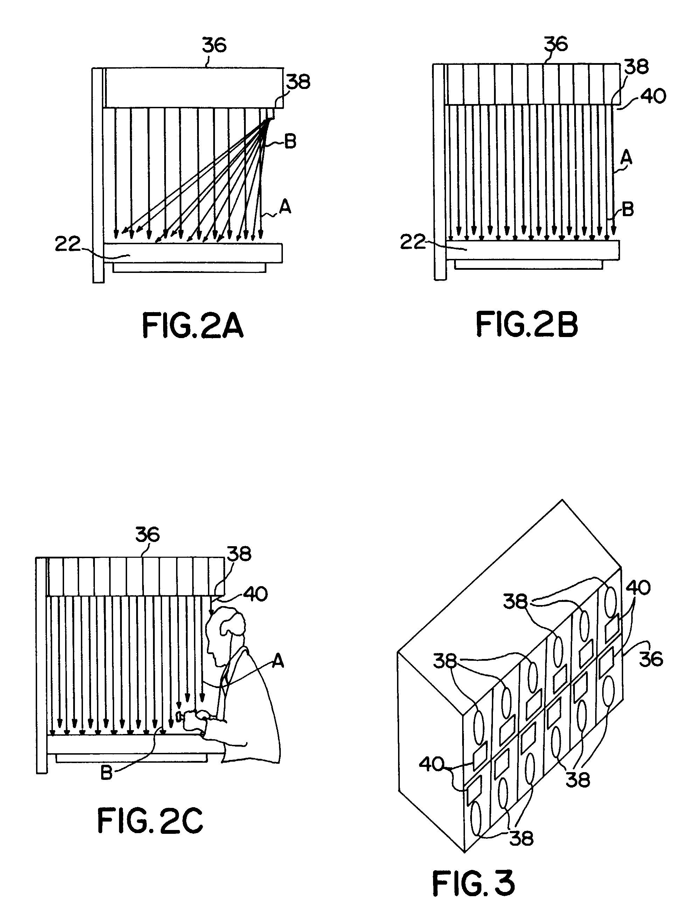

Turning now to FIG.2A there is shown a schematic view of a single proximity sensor 38 used with an infant care center in accordance with the present invention. As shown the pattern of infrared radiation emanating from the heater 36 and impinging upon infant bed 22 is depicted by the arrows A. In FIG.2A, that radiation is not impeded and the pattern of recognition of the proximity sensor 36 is also illustrated and, as indicated, that pattern may be varied as desired by location of the proximity sensor 38 as well as controlling its sensitivity. As shown, however, the intrusion by a caregiver into the pattern of recognition from the proximity sensor 38 would cause the radiant heater 36 to be immediately reduced in intensity, or, possibly turned off altogether. In general, it is preferred to have the intensity of the heater 36 reduced but still remain on so that the infant can continue to be provided with warmth. It is possible, with infants that are larger or older, to turn the heater 36 off for certain periods of time since such infants may not need the constant heat for their well-being.

In FIG. 2B, there is shown a schematic view of a infant warmer wherein a plurality of proximity sensors 38 are utilized along with a plurality of individually controlled radiant heater units 40. In such case, the pattern of recognition of the proximity sensors 38 can be parallel to and coextensive with the pattern of the radiant heat from the heater 36. The pattern of radiant heat from the heater 36 is depicted by the arrows A and the pattern of recognition of the proximity sensor is depicted by the arrows B. In any case, with the FIG. 2B embodiment, the individual heater units 40 may be controlled independently by individual proximity sensors 38 so that it is not necessary to turn the entire heater 36 off or reduce its intensity overall but the corrective action to the heater 36 can be carried out on a localized basis by controlling the intensity of each individual heater unit 40.

In FIG.2C, there is shown a schematic view of the embodiment of FIG. 2B but where a caregiver has entered intrusively into the pattern of the radiant heat and, correspondingly, into the pattern of recognition of the proximity sensor. Thus, when that intrusion is detected, a proximity sensor 38 immediately takes corrective action by signaling the control to the heater 36 to reduce the intensity of the radiation in a localized area to a lower level by affecting the intensity of the infrared energy emitted by one or more individual heater units 40, preferably by simply turning off the closest heater or heaters 36 to the particular proximity sensor or sensors 38 that has recognized the intrusion.

Turning finally to FIG. 3, there is shown an isometric view of a heater 36 having a plurality of heating units 40 and a plurality of proximity sensors 38 as in the FIG. 2B and 2C embodiments. As such, in this embodiment, as the intrusive body enters the pattern of the infrared radiation, the intrusive body may only be detected by one or more of the proximity sensors 38 and which can turn off, or reduce the intensity of, one or more individual heating units 40. Thus, if the person's head enters the pattern of infrared radiation from heater 36, only those individual heating units 40 that are directing the infrared radiation on the persons head are affected and the remaining individual heating units 40 can continue to provide infrared radiation and thus heat to the infant. Thus, the corrective action to the heater 36 can be taken on a localized basis where the protection is provided to the caregiver while the needed heat is still being afforded to the infant.

## Claims

1. An infant care center comprising:
a standing frame member 10;
a generally planar infant bed 22 affixed to said standing frame member 10 and adapted to underlie an infant;
an infrared heater 36 mounted to said standing frame member 10 above said infant bed 22, said infrared heater 36 adapted to radiate electromagnetic radiation in the infrared spectrum at an intensity forming a pattern of infrared radiation directed toward said infant bed 22;
a proximity 38 sensor to detect an intrusion into said pattern of the infrared energy; and
means responsive to the detection of an intrusion by said proximity sensor 38 to change the intensity of the infrared radiation radiated by said infrared heater 36.

2. An infant care center as claimed in Claim 1 wherein said infrared energy is turned completely off in response to the intrusion.

3. An infant care center as claimed in Claim 2 in which the radiation is turned back on when said proximity sensor 38 no longer detects the intrusion.

4. An infant care center as claimed in Claim 1 wherein said infrared radiation is reduced a predetermined amount when the intrusion is detected.

5. An apparatus for heating an infant, said apparatus comprising:
an infant bed 22 adapted to underlie an infant;
an infrared heater assembly 40 mounted above said infant bed 22, said infrared heater assembly 40 adapted to direct electromagnetic radiation in the infrared spectrum at an intensity toward said infant bed 22 in a known pattern,
a proximity sensor 38 adapted to sense the intrusion of a body into said known pattern of infrared radiation directed by said infrared heater assembly 40 toward said infant bed 22; and
a controller means 35 to control said infrared heater assembly 40 to change the intensity of the infrared radiation directed by said infrared heater assembly toward said infant bed 22 when said proximity sensor 38 senses an intrusion.

6. An apparatus for heating an infant as claimed in Claim 5 wherein said controller 35 reduces the intensity of the infrared radiation to a lower intensity when said proximity sensor senses an intrusion.

7. An apparatus for heating an infant as claimed in Claim 5 or 6 wherein said infrared heater assembly 40 comprises a plurality of individual heater units and said proximity sensor 38 comprises a plurality of proximity sensors.

8. An apparatus for heating an infant as claimed in Claim 7 wherein one of said plurality of proximity sensors controls one of said plurality of individual heater units.

9. A method of providing heat to an infant positioned upon an infant bed comprising the steps of:
locating an infrared emitter above the infant bed
directing the infrared radiation emitted from the infrared emitter in a pattern toward the infant bed;
sensing the intrusion of a body into the pattern of radiation directed generally toward infant bed to warm the infant; and
controlling the intensity of the infrared radiation from the infrared emitter in response to the sensed intrusion of the body.

10. A method of providing heat to an infant as claimed in Claim 10 wherein said step of controlling the intensity of the infrared radiation comprises controlling a localized intensity of the infrared radiation emitted by the infrared emitter.
